# EUROPEAN PATENT SPECIFICATION

(11) **EP 1 908 754 B1**
(45) Date of publication and mention of the grant of the patent: **28.01.2009**
(21) Application number: 06019516.1
(22) Date of filing: 19.09.2006
(51) Int. Cl.: C07D 237/32, A61K 31/502, A61P 31/00

(54) **Antibiotic maduraphthalazin salts with delayed release of the active substance and their use**
Antibiotikum Maduraphthalazin-Salze mit verzögerter Wirkstofffreigabe des Wirkstoffs und deren Verwendung
Sels de l'antibiotique maduraphthalazin a action retardée de l'ingredient actif et leur utilisation

(43) Date of publication of application: 09.04.2008
(73) Proprietor: Leibniz-Institut für Naturstoff-Forschung und Infektionsbiologie e.V. Hans-Knöll-Institut, 07745 Jena (DE)
(72) Inventor: Möllmann, Ute, 07749 Jena (DE); Heinemann, Irmgard, 07743 Jena (DE); Vogt, Sebastian, 99092 Erfurt (DE); Schnabelrauch, Matthias, 07749 Jena (DE); Kautz, Armin, 07745 Jena (DE)
(74) Representative: Bock, Gerhard

(56) References cited:
- EP-A2- 1 243 255
- WO-A-00/63181
- WO-A-98/07706

## Description

This invention relates to antibiotic maduraphthalazin salts, which show a low solubility in water and in aqueous media and a delayed release of active substances, and the use of these salts.

Polycyclic maduraphthalazins, 2-carboxyalkyl- and 2-carboxyaryl-substituted maduraphthalazins in form of their N-methyl-D-glucamonium salts present a new type of anti-bacterial substances that are particularly effective against Gram-positive, especially against multiresistant staphylococci (MRSA) and resistant enterococci (W. Witte, C. Cunny, E. Roemer, U. Gräfe, L. Heinisch, U. Möllmann, W. Werner: New polycyclic phthalazine compounds (madura phthalazine derivates) DE 19719522). Due to their great anti-bacterial efficacy the compounds mentioned are especially interesting for the local antibiotic therapy, in particular for the treatment of implant-associated bacterial infections. However, it is to be taken into account that the antibacterially effective carboxyalkylated or carboxyarylated maduraphthalazin salts in form of their N-methyl-D-glucamonium salts are freely soluble compounds in water. Although this property is advantageous for the development of the anti-bacterial effect it is a disadvantage for the local application of the mentioned derivates as a depot, for example in close vicinity to an implant. In the humid milieu of the body water-soluble low-molecular compounds quickly diffuse away from the effective zone and thus they cannot develop their pharmacologic effect at the desired location or they can do it only in a weaker manner. Therefore, the available form of the mentioned freely-soluble maduraphthalzin derivates is not suited for use as a depot system.

Thus, the task of this invention is to develop, on the basis of in water soluble maduraphthalazin salts, depot forms with antibiotic effects that are able to fight successfully against a spectrum of bacterial as broad as possible and that show a controlled release of low quantities of the active substance over a period of several days or weeks in an aqueous milieu. The depot forms shall be used in particular for the local antibiotic therapy of bacterial infections. Moreover, the depot forms to be developed shall provide an effective antibiotic protection of implants over a period of several weeks.

According to the invention, this task is fulfilled by the provision of maduraphthalazin salts that show low solubility in water and consist of an anionic component, which is formed by at least one representative of the group of carboxyalkylated or carboxyarylated maduraphthalazin salts, and of a cationic antibiotic component.
The invention is based on the surprising findings that water-soluble carboxyalkylated or carboxyarylated maduraphthalazin salts in aqueous solution and water-soluble salts of cationic antibiotics form maduraphthalazin salts with low solubility in water in form of precipitates and that in aqueous solution these precipitates exhibit a delayed release of the effective substance over a period of several weeks. The generation of maduraphthalazin salts with low solubility in water by the anionic and cationic components is based on a reciprocal salt exchange.

According to the invention, carboxylalkylated or carboxylarylated maduraphthalazin salts are used as the anionic component.
According to this invention, all the maduraphthalazin salts claimed in DE 19719522 are used as the anionic component.
The use of 2-(4-carboxyphenyl)-maduraphthalazin and/or 2-carboxymethyl-maduraphthalazin and/or 2-carbethoxymethyl-maduraphthalazin and/or 2-(D,L-α-carboxypropyl)-maduraphthalazin salts as an anionic component is particularly advantageous for this invention.

Another feature of this invention is the use of carboxylalkylated or carboxylarylated maduraphthalazin salts in form of water-soluble salts. The use of N-methyl-glucammonium salts of the carboxyalkylated or carboxyarylated maduraphthalazins is particularly preferred.

According to this invention, salts of cationic antibiotics being freely soluble in water are used as the antibiotic component.

A further feature of this invention is that the chlorides, bromides, hydrogen sulfates, sulfates, dihydrogen phosphates, hydrogen phosphates, phosphates, acetates, succinates or lactates of the cationic antibiotics are used as the freely water-soluble salts of said cationic antibiotics.

In the inventive compound, gentamicin, kanamycin, amikacin, clindamicin, neomycin, streptomycin, vancomycin, tetracycline, doxicyclin, oxytetracyclin, rolitetracyclin or mixtures of these antibiotics are used as the cationic antibiotic components.

According to this invention, 0.01 to 10 molar mass parts of the carboxyalkylated or carboxyarylated maduraphthalazins are converted with one mol mass portion of the cationic antibiotic component.
Moreover, it is preferred in this invention that the carboxyalkylated or carboxyarylated maduraphthalazins and the cationic antibiotic component are converted in the stoichiometric mol mass relation according to the existing cationic and anionic groups.

The inventive use is characterized by the fact that the antibiotic maduraphthalazin salts with low solubility in water are integrated into specially shaped bodies, tablets, powder, pellets, gels, films, foils, tubes, threads, textiles, knitwear or fleeces that are used as permanent or temporary implants. These implants can be used as antibiotic depot preparations to fight against bacterial infections at a specified location and they can be used in the precaution against bacterial infections.

It is a further characteristic feature of this invention that the antibiotic maduraphthalazin salts, which have a low solubility in water, are components of coatings that can be applied onto specially shaped bodies, tablets, powder, pellets, gels, films, foils, tubes, threads, textiles, knitwear and fleeces being used as permanent or temporary implants. This refers in particular to coatings on metal implants, on ceramic, glass and glass-ceramic implants, on absorbable and non-absorbable plastic implants as well as on implants being composites of the just mentioned material classes.

A further characteristic feature of this invention is the use of the antibiotic maduraphthalazin salts, which have a low solubility in water, as a sole antibiotic agent or within a mixture with other antibiotics.

In the following, the invention is explained by two examples without being restricted to them.

### Example 1:

### Conversion of gentamicin sulfate with the N-methyl-D-glucamonium salt of 2-carboxymethyl-maduraphthalazin

4.69 mg (0.00634 mmol) of the N-methyl-D-glucamonium salt of 2-carboxymethyl-maduraphthalazin are dissolved in 1 ml water. Then, at room temperature 0.93 mg (0.00127 mmol) gentamicin sulfate AK617 are added to this solution by stirring. A red deposit precipitates. After two hours of stirring at room temperature, this deposit is centrifuged and then washed twice with distilled water. Afterwards, the product is dried in vacuum at room temperature till the mass constant is reached. In this way, 3.6 mg of a red solid are obtained.

Wave numbers of 12 absorption-bands detectable in the FT-IR spectrum, which shows clearly marked peaks. These are in detail [1/cm]: 2946,6; 1610,1; 1587,7; 1434,5; 1407,5; 1374,7; 1351,7; 1304,2; 1265,9; 1202,9; 1051,0; 1039,3.

### Example 2:

1.9 mg (0.00257 mmol) of the N-methyl-D-glucamonium salt of the 2-carboxymethyl-maduraphthalazin are converted with 0.232 mg (0.000513 mmol) gentamicin sulfate AK 628 in aqueous solution at room temperature within two hours. The release of the active substance has been examined by adding 1.5 mg of the produced salt to 2 ml distilled water. The sample has been stored at 37°C. The excess portion has been removed daily. Then, the samples have been refilled anew with 2 ml fresh distilled water. The active substance release has been determined by means of a microbial agar-diffusion test by using the test organism *Bacillus subtilis* ATCC 6633 and by using a synthetic nutrient agar. The Agar plates has been incubated at 37°C over a period of 18 hours. The diameter of the inhibition zones has been measured after the incubation.

**Table 1. Inhibition zone diameter of Bacillus subtilis ATCC6633 as a function of the release time.**

| Time (d) | Inhibition zone diameter (mm) |
|---|---|
| 1 | 27.0 |
| 2 | 22.0 |
| 3 | 21.0 |
| 9 | 17.5 |
| 20 | 18.0 |
| 45 | 22 |
| 51 | 20 |
| 65 | 18 |
| 74 | 18 |
| 100 | 19 |
| 151 | 17 |
| 162 | 16 |

## Claims

1. Antibiotic maduraphthalazin salts showing low solubility in water and a delayed release of the active substance, which are formed by an anionic component consisting of at least one representative of the group of carboxyalkylated or carboxyarylated maduraphthalazin salts, and of a cationic antibiotic component.

2. Antibiotic maduraphthalazin salts showing low solubility in water and a delayed release of the active substance according to claim 1, wherein 2-(4-carboxyphenyl)-maduraphthalazin and/or 2-carboxymethyl maduraphthalazin and/or 2-carbethoxymethyl-maduraphthalazin and/or 2-(D,L-α-carboxypropyl)-maduraphthalazin are used as the anionic component in form of salts that are soluble in water.

3. Antibiotic maduraphthalazin salts showing low solubility in water and a delayed release of the active substance according to claim 2, wherein N-methyl-glucamonium salts of 2-(4-carboxyphenyl)-maduraphthalazin and/or 2-carboxymethyl-maduraphthalazin and/or 2-carbethoxymethyl-maduraphthalazin and/or 2-(D,L-α-carboxypropyl)-maduraphthalazin are used as salts that are soluble in water.

4. Antibiotic maduraphthalazin salts showing low solubility in water and a delayed release of the active substance according to claim 1, wherein salts of the antibiotics gentamicin, kanamycin, amikacin, clindamicin, neomycin, streptomycin, vancomycin, tetracyclin, doxicyclin, oxytetracyclin, rolitetracyclin or mixtures of these antibiotic salts that are freely soluble in water are used as a cationic antibiotic component.

5. Antibiotic maduraphthalazin salts showing low solubility in water and a delayed release of the active substance according to claim 4, wherein as the antibiotic salts being freely soluble in water their chlorides, bromides, hydrogen sulfates, sulfates, dihydrogen phosphates, hydrogen phosphates, phosphates, acetates, succinates or lactates are used.

6. Antibiotic maduraphthalazin salts showing low solubility in water and a delayed release of the active substance according to one of the claims 1 through 5, wherein for their production 0.01 to 10 mol mass portions of the carboxyalkylated or carboxyarylated maduraphthalazin or maduraphthalazin compound are converted with one mol mass portion of the cationic antibiotic component.

7. Antibiotic maduraphthalazin salts showing low solubility in water and a delayed release of the active substance according to one of the claims 1 through 6, wherein for their production the carboxyalkylated or carboxyarylated maduraphthalazin or maduraphthalazin compound and the cationic antibiotic component are converted in a stoichiometric mol mass relation referred to the existing cationic and anionic groups.

8. Use of an antibiotic low-water-soluble maduraphthalazin salt according to one of the claims 1 through 7 for manufacture of permanent or temporary implants that are used as antibiotic depot preparations to fight against bacterial infections at a specific location or to prevent infections and that are integrated into the body in form of compact or porous specially shaped bodies, powders, pellets, gels, films, foils, tubes, threads, textiles, knitwear or fleeces.

9. Use of an antibiotic, low-water-soluble maduraphthalazin salt according to one of the claims 1 through 8 for manufacture of coating for permanent or temporary implants that are integrated in form of compact or porous specially shaped bodies, powders, pellets, gels, films, foils, tubes, threads, textiles, knitwear or fleeces.

10. Use of an antibiotic, low-water-soluble maduraphthalazin salt according to one of the claims 1 through 9 for manufacture of coating or as an element of a coating on metal implants, on ceramic, glass and glass-ceramic implants, on absorbable and non-absorbable plastic implants as well on as implants being composites of the just mentioned material classes.

11. Use of an antibiotic, low-water-soluble maduraphthalazin salt according to one of the claims 1 through 10, for manufacture of a combination comprising the maduraphthalazin salt and other antibiotics that have a low solubility and/or are freely soluble in water.

## Patentansprüche

1. Antibiotische in Wasser gering lösliche Maduraphthalazinsalze mit verzögerter Wirkstofffreisetzung, **dadurch gekennzeichnet, dass** sie aus einer anionischen Komponente, die aus mindestens einem Vertreter der Gruppe der carboxyalkylierten oder carboxyarylierten Maduraphthalazinsalze gebildet ist und einer kationischen Antibiotikumkomponente aufgebaut sind.

2. Antibiotische in Wasser gering lösliche Maduraphthalazinsalze mit verzögerter Wirkstofffreisetzung nach Anspruch 1, **dadurch gekennzeichnet, dass** 2-(4-Carboxyphenyl)-Maduraphthalazin und/oder 2-Carboxymethyl-Maduraphthalazin und/oder 2-Carbethoxymethyl-Maduraphthalazin und/oder 2-(D,L-α-Carboxypropyl)-mMaduraphthalazin als anionische Komponente in Form wasserlöslicher Salze eingesetzt werden.

3. Antibiotische in Wasser gering lösliche Maduraphthalazinsalze mit verzögerter Wirkstofffreisetzung nach Anspruch 2, **dadurch gekennzeichnet, dass** N-Methyl-Glucammoniumsalze von 2-(4-Carboxyphenyl)-Maduraphthalazin und/oder 2-Carboxymethyl-Maduraphthalazin und/oder 2-Carbethoxymethyl-Maduraphthalazin und/oder 2-(D,L-α-Carboxypropyl)-Maduraphthalazin als wasserlösliche Salze verwendet werden.

4. Antibiotische in Wasser gering lösliche Maduraphthalazinsalze mit verzögerter Wirkstofffreisetzung nach Anspruch 1, **dadurch gekennzeichnet, dass** leicht wasserlösliche Salze der Antibiotika Gentamicin, Kanamycin, Amikacin, Clindamycin, Neomycin, Streptomycin, Vancomycin, Tetracyclin, Doxicyclin, Oxytetracyclin, Rolitetracyclin oder Mischungen dieser Antibiotikasalze als kationische Antibiotikumkomponente verwendet werden.

5. Antibiotische in Wasser gering lösliche Maduraphthalazinsalze mit verzögerter Wirkstofffreisetzung nach Anspruch 4, **dadurch gekennzeichnet, dass** als leicht wasserlösliche Antibiotikumsalze deren Chloride, Bromide, Hydrogensulfate, Sulfate, Dihydrogenphosphate, Hydrogenphosphate, Phosphate, Acetate, Succinate oder Lactate eingesetzt werden.

6. Antibiotische in Wasser gering lösliche Maduraphthalazinsalze mit verzögerter Wirkstofffreisetzung nach einem der Ansprüche 1 bis 5, **dadurch gekennzeichnet, dass** zu deren Herstellung 0,01 bis 10 Molmengenteile des carboxyalkylierten oder carboxyarylierten Maduraphthalazins oder Maduraphthalazingemischs mit einem Molmengenteil der kationischen Antibiotikumkomponente umgesetzt werden.

7. Antibiotische in Wasser gering lösliche Maduraphthalazinsalze mit verzögerter Wirkstofffreisetzung nach einem der Ansprüche 1 bis 6, **dadurch gekennzeichnet, dass** zu deren Herstellung das carboxyalkylierte oder carboxyarylierte Maduraphthalazin oder Maduraphthalazingemisch und die kationische Antibiotikumkomponente im stöchiometrischen Molmengenverhältnis bezogen auf vorhandene kationische und anionische Gruppen umgesetzt werden.

8. Verwendung eines antibiotischen in Wasser gering löslichen Maduraphthalazinsalzes nach einem der Ansprüche 1 bis 7 zur Herstellung permanenter oder temporärer Implantate, die als antibiotische Depotpräparate zur lokalen Bekämpfung von bakteriellen Infektionen oder zur Infektionsprophylaxe eingesetzt werden und als kompakte oder poröse Formkörper, Pulver, Granulate, Gele, Filme, Folien, Schläuche, Fäden, Gewebe, Gewirke oder Vliese in den Körper eingebracht werden.

9. Verwendung eines antibiotischen in Wasser gering löslichen Maduraphthalazinsalzes nach einem der Ansprüche 1 bis 8 zur Herstellung einer Beschichtung für permanente oder temporäre Implantate, die in Form von kompakten oder porösen Formkörpern, Pulvern, Granulaten, Gelen, Filmen, Folien, Schläuchen, Fäden, Geweben, Gewirken oder Vliesen eingebracht werden.

10. Verwendung eines antibiotischen in Wasser gering löslichen Maduraphthalazinsalzes nach einem der Ansprüche 1 bis 9 zur Herstellung einer Beschichtung oder eines Bestandteils einer Beschichtung auf Metallimplantaten, Keramik-, Glas- und Glaskeramikimplantaten, resorbierbaren und nichtresorbierbaren Kunststoffimplantaten sowie auf Implantaten, die Verbundwerkstoffe der vorstehend genannten Materialklassen darstellen.

11. Verwendung eines antibiotischen in Wasser gering löslichen Maduraphthalazinsalzes nach einem der Ansprüche 1 bis 10, **dadurch gekennzeichnet, dass** das antibiotische in Wasser gering lösliche Maduraphthalazinsalz zur Herstellung einer Kombination verwendet wird, die aus dem Maduraphthalazinsalz und anderen in Wasser gering löslichen und/oder leicht löslichen Antibiotika besteht.

## Revendications

1. Sels de l'antibiotique maduraphtalazin à faible solubilité dans l'eau et à action retardée de l'ingrédient actif sont **caractérisés en ce qu'**ils sont formés d'un composant anionique qui se compose au moins d'un représentant du group des sels maduraphtalazins alcylés ou arylés à carboxyle et d'un composant d'antibiotique cationique.

2. Sels de l'antibiotique maduraphtalazin à faible solubilité dans l'eau et à action retardée de l'ingrédient actif suivant la revendication 1 sont **caractérisés en ce que** maduraphtalazin de 2-(4-carboxyphényle) et/ou maduraphtalazin de 2-carboxyméthyle et/ou maduraphtalazin de 2-carbethoxyméthyle et/ou maduraphtalazin de 2-(D,L-α-carboxypropyle) sont utilisés comme composant anionique sous forme des sels solubles dans l'eau.

3. Sels de l'antibiotique maduraphtalazin à faible solubilité dans l'eau et à action retardée de l'ingrédient actif suivant la revendication 2 sont **caractérisés en ce que** les sels de N-méthyle-glucammonium du maduraphtalazin de 2-(4-carboxyphényle) et/ou maduraphtalazin de 2-carboxyméthyle et/ou maduraphtalazin de 2-carbethoxyméthyle et/ou maduraphtalazin de 2-(D,L-α-carboxypropyle) sont utilisés comme sels solubles dans l'eau.

4. Sels de l'antibiotique maduraphtalazin à faible solubilité dans l'eau et à action retardée de l'ingrédient actif suivant la revendication 1 sont **caractérisés en ce que** des sels à bonne solubilité dans l'eau des antibiotiques gentamicine, kanamycine, amikacine, clindamycine, néomycine, streptomycine, vancomycine, tétracycline, doxicycline, oxytétracycline, rolitétracycline ou des mixtures de ce sels antibiotiques sont utilisés comme composant d'antibiotique cationique.

5. Sels de l'antibiotique maduraphtalazin à faible solubilité dans l'eau et à action retardée de l'ingrédient actif suivant la revendication 4 sont **caractérisés en ce que** leurs chlorures, bromures, sulfates d'hydrogène, sulfates, dihydrogènephosphates, hydrogènephosphates, phosphates, acétates, succinates ou lactates sont utilisés comme sels de l'antibiotique à bonne solubilité dans l'eau.

6. Sels de l'antibiotique maduraphtalazin à faible solubilité dans l'eau et à action retardée de l'ingrédient actif suivant une des revendications 1 à 5 sont **caractérisés en ce que** pour leur production, des portions de masse molaire de 0,01 à 10 de maduraphtalazin alcylé ou arylé à carboxyle ou de la mixture maduraphtalazin sont convertis avec un portion de masse molaire du composant d'antibiotique cationique.

7. Sels de l'antibiotique maduraphtalazin à faible solubilité dans l'eau et à action retardée de l'ingrédient actif suivant une des revendications 1 à 6 sont **caractérisés en ce que** pour leur production, le maduraphtalazin alcylé ou arylé à carboxyle ou la mixture maduraphtalazin et le composant d'antibiotique cationique sont convertis avec un rapport molaire stoechiométrique se référant aux groupes existants d'antibiotique cationique et anionique.

8. Utilisation d'un sel de l'antibiotique maduraphtalazin à faible solubilité dans l'eau selon une des revendications 1 à 7 pour produire des implants permanents ou temporaires, qui sont utilisés comme préparation-retards antibiotiques pour la lutte locale contre les infections bactérielles ou pour la prophylaxe d'infections et qui sont introduits dans le corps comme corps de moule compacts ou poreux, poudres, granulés, gels, films, feuilles, tuyaux flexibles, fils, tissus, maillages ou toisons.

9. Utilisation d'un sel de l'antibiotique maduraphtalazin à faible solubilité dans l'eau selon une des revendications 1 à 8 pour produire un revêtement pour les implants permanents ou temporaires, qui sont introduits comme corps de moule compacts ou poreux, poudres, granulés, gels, films, feuilles, tuyaux flexibles, fils, tissus, maillages ou toisons.

10. Utilisation d'un sel de l'antibiotique maduraphtalazin à faible solubilité dans l'eau selon une des revendications 1 à 9 pour produire un revêtement ou un composant d'un revêtement sur des implants métalliques, des implants céramiques, vitrocéramiques et de verre, des implants en matière plastique résorbables et non résorbables ainsi que sur des implants représentant les matériaux composites formés des classes de matériaux dénommées ci-dessus.

11. L'utilisation d'un sel de l'antibiotique maduraphtalazin à faible solubilité dans l'eau selon une des revendications 1 à 10 est **caractérisée en ce que** le sel de l'antibiotique maduraphtalazin à faible solubilité dans l'eau est utilisé pour produire une combinaison qui se compose du sel de l'antibiotique maduraphtalazin et d'un autre antibiotique à faible et/ou bonne solubilité dans l'eau.
